(19) Europäisches Patentamt
European Patent Office
Office européen des brevets

(11) Publication number: **0 499 322 A1**

# (12) EUROPEAN PATENT APPLICATION

(21) Application number: **92200341.3**

(22) Date of filing: **07.02.92**

(30) Priority: **15.02.91 EP 91200319**

(43) Date of publication of application:
**19.08.92 Bulletin 92/34**

(84) Designated Contracting States:
**PT**

(51) Int. Cl.[5]: **C08B 37/16**, A61K 47/48

(71) Applicant: **JANSSEN PHARMACEUTICA N.V.**
**Turnhoutseweg 30**
**B-2340 Beerse(BE)**

(72) Inventor: **Szejtli, J zsef**
**Rákos u. 8**
**H-1028 Budapest(HU)**
Inventor: **Jicsinszky, Lászl**
**Kosárfon u. 28**
**H-1191 Budapest(HU)**

(74) Representative: **Wante, Dirk**
**Janssen Pharmaceutica N.V., Patent Department, Turnhoutseweg 30**
**B-2340 Beerse(BE)**

## (54) (Carboxyl)alkyloxyalkyl derivatives of cyclodextrins.

(57) Clyclodextrin ethers or mixed ethers wherein one or several hydroxy groups of the cyclodextrin moiety are etherified with mono- or dihydroxyalkyl and in the case of mixed ethers, one or more of the remaining hydroxy groups of the cyclodextrin moiety are further etherified with (carboxyl)alkyl or (derivatized carboxyl)alkyl, and wherein one or more of the hydroxy groups of said mono- or dihydroxyalkyl groups are further etherified with (carboxyl)alkyl or (derivatized carboxyl) alkyl; the alkylcarbonylated derivatives thereof; the non-toxic metal and amine salt forms thereof; and the stereochemically isomeric forms thereof. Process for preparing said cyclodextrin derivatives. Cyclodextrin derivatives obtainable by said process. Complexes and pharmaceutical compositions comprising said cyclodextrin derivatives.

EP 0 499 322 A1

## Background of the invention

Cyclodextrins are cyclic carbohydrates consisting of 6, 7 or 8 glucose units which are designated respectively $\alpha$, $\beta$, and $\gamma$-cyclodextrin. Due to their unique spatial configuration, cyclodextrins can form complexes with a variety of substances, including active substances such as drugs. In many cases, complexation with cyclodextrins changes the characteristics of an active substance dramatically : virtually insoluble active substances become bioavailable and (physico)chemically labile active substances are stabilized. However, the solubility of most drug-cyclodextrin complexes is still not satisfactory for application in injectable solutions. Moreover, the non-derivatized cyclodextrins - in particular $\beta$-cyclodextrin - show nephrotoxic effects upon parenteral administration. Derivatization of cyclodextrins leads in most cases to more soluble cyclodextrin derivatives. A number of such derivatives are described in U.S. Patent 3,459,731, in EP-A-149,197 and EP-A-197,571. These derivatives show limited haemolytic properties and can be administered parenterally. Related aminoalkyl-, or mixed (alkyl-, hydroxyalkyl-, or carboxyalkyl) cyclodextrin ether derivatives are known from EP-A-146,841 and EP-A-147,685.

It is known that the increase of the number and bulkyness of substituents on the ring of the cyclodextrin torus, decreases the accessibility of the cyclodextrin cavity. In this respect a relatively low degree of substitution apparently would be more advantageous. On the other hand, increasing the degree of the substitution improves the solubility, whereas the introduction of polar groups seems to reduce haemolytic activity. Hence, it would seem advantageous to increase the number and the polar character of the substituents on the cyclodextrin torus, should it not result in a decreased guest molecule solubilizing capacity because of a partial blocking of the cavity of the cyclodextrin torus.

It now has been found that the particular cyclodextrin derivatives according to the present invention combine the advantages of the presence of polar substituents while at the same time keeping an acceptable degree of accessibility to the cyclodextrin cavity.

## Description of the invention

The present invention is concerned with cyclodextrin ethers or mixed ethers wherein one or several hydroxy groups of the cyclodextrin moiety are etherified with mono-or dihydroxyalkyl and in the case of mixed ethers, one or more of the remaining hydroxy groups of the cyclodextrin moiety are further etherified with (carboxyl)alkyl or (derivatized carboxyl)alkyl, characterized in that one or more of the hydroxy groups of said mono- or dihydroxyalkyl groups are further etherified with (carboxyl)alkyl or (derivatized carboxyl) alkyl; the alkylcarbonylated derivatives thereof; the non-toxic metal and amine salt forms thereof; and the stereochemically isomeric forms thereof.

In the foregoing and hereinafter, the term cyclodextrin defines $\alpha$, $\beta$, and $\gamma$-cyclodextrin, $\gamma$- and in particular $\beta$-cyclodextrin being preferred. The term "alkyl" refers to monovalent, saturated straight or branch chained hydrocarbon radicals having from 1 to 4 carbon atoms, such as, for example methyl, ethyl, propyl, 1-methylethyl, butyl, 1-methylpropyl, 2-methylpropyl and 1,1-dimethylethyl; the term "alkyl" used in composite definitions such as "mono- or dihydroxyalkyl", "-(carboxyl)alkyl" and "(derivatized carboxyl)alkyl" defines bivalent or trivalent, saturated, straight or branch chained hydrocarbon radicals having from 1 to 4 carbon atoms, such as, for example methylene, 1,1-ethanediyl, 1,2-ethanediyl, 1,2-propanediyl, 1,3-propanediyl, 1,4-butanediyl, 1,2,3-propanetriyl, 1,2,3-butanetriyl and the like. Preferably, the alkyl moiety in the mono- or dihydroxyalkyl groups in the above defined cyclodextrin ethers and mixed ethers contains at least 2 carbon atoms between any two ether or hydroxyl oxygen atoms, examples of such alkyl moieties being 1,2-ethanediyl, 1,2-propanediyl, 1,2-butanediyl, 1,3-propanediyl, 1,3-butanediyl and the like.

The term "etherified with mono- or dihydroxyalkyl" means that a hydroxy group of the cyclodextrin moiety may be etherified with one or more hydroxy- or dihydroxyalkyl groups. Indeed a hydroxy- or dihydroxyalkyl group can be further etherified thus forming oligomeric hydroxyalkylethers of the type hydroxyalkyl-polyoxyalkyl.

"Alkylcarbonylated derivatives" refers to cyclodextrin derivatives as defined above wherein one or more hydroxy groups are acylated with $C_{1-4}$alkylcarbonyl groups, in particular with acetyl groups.

The invention also comprises the non-toxic metal and amine salt forms of the above-defined cyclodextrin derivatives derived therefrom by treatment with organic or inorganic bases such as amines, alkali metal bases and alkaline earth metal bases, or quaternary ammonium bases. These salt forms can conveniently be obtained by treating the present cyclodextrin derivatives with appropriate inorganic or organic bases. Conversely the salt form can be converted by treatment with acid into the free acidic form.

Preferred salt-forming alkali metal hydroxides and alkaline earth metal hydroxides are the hydroxides of lithium, sodium, potassium, magnesium or calcium, more particularly sodium or potassium hydroxide. Examples of appropriate salt-forming

amines are the primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, pyridine, quinoline, isoquinoline and quinuclidine. Preferred amines are ethylamine, propylamine, diethylamine or triethylamine and more particularly isopropylamine, diethanolamine or 1,4-diazabicyclo[2.2.2]octane. Examples of quaternary ammonium salts generally comprise cations arising from ammonium hydroxides or ammonium halogenide salts, for example, the ammonium, tetramethylammonium, tetraethylammonium, trimethylethylammonium or trimethylbenzylammonium cation.

Derivatized carboxyl as used in the aforementioned definitions comprises carboxyl derivatives obtained from the carboxyl group by simple derivatization reactions such as esterification and amidation reactions. Said derivatives comprise amides, mono- or dialkylamides, cyclic alkylamides and alkyl esters, wherein alkyl represents a monovalent, saturated straight or branch chained hydrocarbon radical having from 1 to 4 carbon atoms, preferably having 1 or 2 carbon atoms. Examples of such derivatives are for example the methyl, ethyl, propyl and the like esters, amides, e.g. methylamide, ethylamide, dimethylamide, diethylamide and the like. Cyclic alkylamides refers to radicals

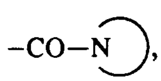

wherein

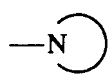

is morpholinyl, piperidinyl, pyrrolidinyl or piperazinyl.

In the cyclodextrin derivatives defined above or hereinafter, the MS of the hydroxyalkyl or dihydroxyalkyl substituents in particular is in the range from $\frac{1}{8}$, $\frac{1}{7}$ or $\frac{1}{6}$ to 10, the minimum value of $\frac{1}{8}$ being for γ-cyclodextrin derivatives, $\frac{1}{7}$ being for β-cyclodextrin derivatives and $\frac{1}{6}$ being for α-cyclodextrin derivatives. Preferably the MS ranges from about 0.3 to 3, or from 0.3 to 1.5. More preferably, the MS is between 0.3 and 0.8 or between 0.35 and 0.5, and most preferably, the MS is bout 0.4 Preferably the DS ranges from about 0.2 to 2, from 0.2 to 1.5. More preferably the DS is between 0.2 and 0.7, or between 0.35 and 0.5, and most preferably the DS is about 0.4. The degree of substitution of the (carboxyl)alkyl or (derivatized carboxyl)alkyl group bound to the mono- or dihydroxyalkyl ether substituents ranges from $\frac{1}{8}$, $\frac{1}{7}$, $\frac{1}{6}$ to 3 and in particular from $\frac{1}{8}$, $\frac{1}{7}$, $\frac{1}{6}$ to 1.

MS as referred to hereinabove or hereinafter refers to the "average Molar Substitution" which is used to indicate the number of mono- or dihydroxyalkyl substituents, is defined as the average number of moles of the substituting agent per glucose unit. In the same way the descriptor DS refers to "average Degree of Substitution" and refers to the average number of substituted hydroxy functions per glucose unit. These definitions comply with the same used in EP-A-0,197,571 and EP-0,149,197. DS as related to the (carboxyl)alkyl or (substituted carboxyl)alkyl substituents defines the average number of such substituents per glucose unit, i.e. the total number of such substituents in the total cyclodextrin molecule divided by the number of glucose units in said molecule.

In particular, the present invention concerns cyclodextrin ethers or mixed ethers wherein one or several hydroxy groups of the cyclodextrin moiety are etherified with a substituent of formula

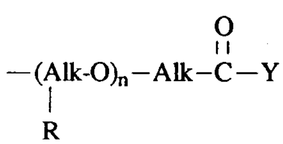 (a)

wherein R is hydrogen, hydroxy, $-O-(Alk-O)_n-H$ or $-O-(Alk-O)_n-Alk-C(=O)-Y$,

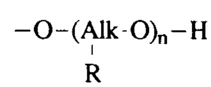

or

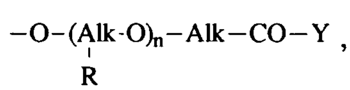

and wherein R may be present on one or more of the -Alk-O- moieties;

each Alk independently is $C_{1-4}$alkanediyl; and where Alk is substituted with R, Alk is $C_{2-4}$alkanetriyl;

each n independently is the integer 1 to 10; Y is $OR^1$ or $NR^2_2$ wherein $R^1$ and $R^2$ each independently are hydrogen or $C_{1-4}$alkyl, or both $R^2$ combined with the nitrogen bearing these substituents may form a morpholinyl, piperidinyl, pyrrolidinyl or piperazinyl ring;

and one or more of the remaining hydroxy groups of the cyclodextrin moiety may be further etherified with a radical -(Alk-O)$_n$-H or -Alk-CO-Y; including the alkylcarbonylated derivatives, salts and stereoisomers thereof, as defined hereinabove.

Particular radicals of formula (a) are, for example :

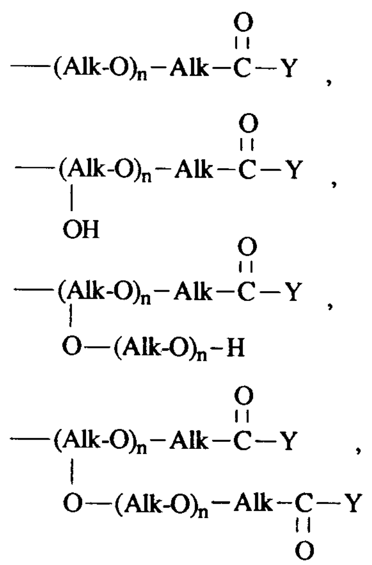

the first of the above radicals being preferred.
In these radicals Y is $NH_2$ or $OR^1$, wherein $R^1$ in particular is hydrogen or $C_{1-4}$alkyl, preferably hydrogen, methyl or ethyl;
n in particular is 1 to 5, more in particular 1 to 3;
Alk in the -(Alk-O)$_n$- moieties in particular is $-CH_2-CH_2-$, $-CH_2-CH(CH_3)-$, $-CH_2-CH_2-CH_2-$,

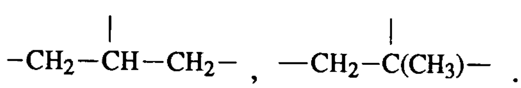

Alk as used in Alk-CO-Y in particular is $CH_2$, $C_2H_4$, $CH(CH_3)$, $-CH_2-CH_2-CH_2-$, $-CH_2-CH_2-CH_2-CH_2-$.

In the preferred cyclodextrin derivatives of the invention, (a) represents $-(CH_2-CH_2-O)_n-Alk'-COOH$,

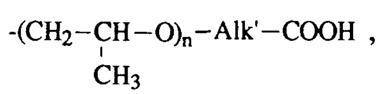

$-(CH_2-CH_2-CH_2-O)-Alk'-COOH$, wherein Alk' is $-CH_2-$ or $-CH_2-CH_2-$; the salts thereof, or the methyl, ethyl ester derivatives, or the mono- or dimethyl- or ethylamide derivatives thereof.

A further aspect of the present invention comprises a novel process for making the cyclodextrin derivatives described hereinabove.

This process comprises O-alkylating cyclodextrin ethers or mixed ethers wherein one or several hydroxy groups of the cyclodextrin moiety are etherified with mono- or dihydroxyalkyl and in the case of mixed ethers, one or more of the remaining hydroxy groups of the cyclodextrin moiety are further etherified with (carboxyl)alkyl or (derivatized carboxyl)alkyl, with a (leaving group substituted) $C_{1-4}$alkyl carboxylic acid or acid derivative or with a $\alpha$, $\beta$-$C_{2-4}$alkenyl carboxylic acid or acid derivative. The aforementioned leaving group in particular is a sulfonyloxy group such as a mesylate, tosylate, brosylate and the like, or a halo group, iodo or preferably chloro or bromo. Under appropriate conditions it has proven possible to etherify selectively the hydroxyalkyl or dihydroxyalkyl substituents while leaving all or most of the non-substituted hydroxyls of the cyclodextrin ring unreacted. In this way, carboxylalkyloxyalkyl or -polyoxyalkyl ethers are formed. By varying the amount of the carboxylic acid reagent, the degree of substitution as well as the substitution site can be modified.

The starting cyclodextrin ethers or mixed ethers are reacted with (leaving group substituted) alkyl carboxylic acids or $\alpha,\beta$-alkenyl carboxylic acids (i.e. acrylic acid derivatives), preferably under atmospheric pressure, and at moderately elevated temperatures, in particular in the range 20-90 °C. The reaction preferably is conducted in the presence of a suitable base, in aqueous solution or in organic solvent suspension. Suitable bases comprise, depending on the solvent, alkali- or alkaline earth metal hydroxides, alkoxides (such as methoxides, ethoxides), hydrides or amides (such as sodium hydride, calcium amide, sodium amide). Good results are obtained when using hydroxides. An amount of the (leaving group substituted) alkyl carboxylic acid or of the acrylic acid derivative is added, which is sufficient to accomplish the desired amount of addition to the substituted cyclodextrin molecule. It has been found that the amount of base influences the yield of the reaction and the site where the substitution occurs. Small amounts of base, and in particular of alkali- or alkaline earth metal hydroxydes seem to favour O-alkylation of primary hydroxyls. Larger quantities of base seem to promote substitution at secondary hydroxyls. With very high excess of base, random substitutions are obtained. Usually, amounts of base below 5 equivalents, more in particular below 3 equivalents and still more particular below 1 equivalent, seriously reduce the yield of the reaction.

The course of the reaction may be followed by TLC and is deemed to be terminated when no changes are detected. Upon termination of the reaction, the base may optionally be neutralized by addition of a suitable acid, such as an inorganic acid, e.g. diluted hydrochloric acid, sulfuric acid,or of carbon dioxide. After neutralization, the aqueous

solution containing the cyclodextrin end product may optionally be acidified by addition of a suitable acid such as e.g. concentrated hydrochloric acid or sulfuric acid. Preferably sulfuric acid is used since it is easier to handle and usually is less contaminated with metals compared to hydrochloric acid.
The reaction products may be purified by dissolving the crude material is a $C_{1-4}$alkanol, preferably in ethanol, if desired by refluxing the crude product with ethanol, whereupon the solid residue is filtered and the liquid phase is evaporated and lyophilized. The salt contents of the thus obtained material ranges from about 2 to 5%. Further removal of inorganic salts from the reaction products may be performed following art-known purification techniques e.g. fractionated precipitaton, chromatography, ion-exchange chromatography and dialysis. For example, the reaction products may be further purified by dissolving said products in distilled water and dialysing the mixture through a membrane against an amount of stirred distilled water over a sufficient period of time e.g. 6 to 10 hours, preferably 8 hours. After lyophilizing, the salt content of the reaction product is reduced below 0.5% and in general is approximately 0.2%."

Particular haloalkyl carboxylic acids or acrylic acid derivatives which can be used in the above process comprise the chloro- or bromo$C_{1-4}$alkyl carboxylic acids such as chloro- or bromacetic acid, chloro- or bromopropionic acid, chloro- or bromobutyric acid or the $C_{1-4}$alkyl esters thereof; or acrylic acid or $C_{1-3}$alkyl acrylic acids such as methacrylic acid; further acrylic acid derivatives comprise acrylic or $C_{1-3}$alkyl acrylic amide; or N-$C_{1-4}$alkyl or N,N-di$C_{1-4}$alkyl acrylic or $C_{1-3}$alkyl acrylic amide.

The alkylcarbonylated forms of the cyclodextrin derivatives as defined above can be obtained by the reaction of the appropriate starting cyclodextrin derivatives with acid anhydrides or halides, in particular chlorides or bromides, optionally in the presence of a suitable base such as pyridine or a trialkylamine e.g. triethylamine, or a suitable Lewis acid, at an appropriate temperature, preferably between 10-100 °C. Particular acid anhydrides or halides for this reaction comprise for example acetic, propionoic, or butyric anhydride or acetyl, propionyl or butyryl chloride. The aforementioned Lewis acids comprise anhydrous $ZnCl_2$, $FeCl_3$ or $AlCl_3$; or $BF_3$-etherate.

The carboxylalkyl groups in the cyclodextrin derivatives of the present invention can be converted to ester groups by reaction with ester forming agents such as methyl iodide or with dialkyl sulfates or carbonates, optionally in the presence of a suitable base. Suitable bases for this purpose comprise alkali or alkaline earth metal hydroxydes, organic bases such as pyridine or trialkylamines, e.g. triethylamine. During the reaction, the base optionally may be neutralized, e.g. by addition of a suitable acid.

The starting mono- or dihydroxy alkyl substituted cyclodextrins are known and may be prepared according to procedures described in for example EP-A-0,197,571 or US-4,764,604, or analogous procedures.

Of particular utility in the preparation of the cyclodextrin derivatives of the invention are the $\alpha$, $\beta$ -or $\gamma$-cyclodextrin ethers or mixed ethers wherein one or more hydroxy groups are etherified with a hydroxyethyl, 2-hydroxypropyl, 3-hydroxypropyl, or a 2,3-dihydroxypropyl group, also including the hydroxyalkyl polyoxyalkyl forms thereof such as hydroxyethoxyethyl and the like.
The cyclodextrin derivatives according to the present invention can be used to form complexes with a wide variety of chemical substances, either showing biological or therapeutic activity, or not. Examples of the latter comprise dyestuffs, agents for use in photography such as photographic colour couplers, fragrances, food additives and the like. In particular the present cyclodextrin derivatives can be used to form complexes of substances such as described in Hungarian Patent No. 181,703, US Pat. No. 4,535,152, EP-A-149,197 and EP-A-197,571. Preferably, the ingredients to be complexed show biological activity and comprise phytopharmaceuticals, herbicides, insecticides, fungicides and the like or said ingredients show therapeutical activity, and comprise active ingredients for human or veterinary use, i.e. drugs.

In said complexes, the molar ratio of cyclodextrin : active compound is in the range from about 1:1 to about 20:1, in particular form, about 1:1 to about 15:1, more in particular from about 1:1 to 10:1 or 2:1 to 15:1 or 2:1 to 10:1, depending on the active compound. Thus, in general, the complex is prepared by dissolving the cyclodextrin derivative in water and adding the active compound to this solution, optionally under vigorous stirring and at a temperature in the range of 10 °C to 50 °C, in particular in a range of 15 °C to 30 °C, preferably at room temperature.

Complexes with an appropriate guest molecule may also be formed by *in situ* formation or any other art-known method. Stability and solubility measurements of these complexes can be performed by known methods (see J. Szejtli, Cyclodextrin Technology, Kluwer Academic Publishers, 1988, Chapter 2).

The cyclodextrin derivatives of the present invention, although having quite voluminous polar substituents, do not show a restricted accessibility for guest molecules. Further, they show an increased capacity to solubilize all kinds of chemical substances, particularly those having polar groups,

and more in particular those with basic groups. They also show an increased stabilizing effect on chemically or physico-chemically labile substances. Further, they also possess beneficial toxicological properties, e.g. a limited haemolytic effect.

The complexes of chemical substances with the present cyclodextrin derivatives can be mixed or formulated with other ingredients in the usual manner. In case of active ingredients for human or veterinary use, the complexes may be formulated in the commonly known application forms for oral, parenteral, topical, rectal or vaginal applications, such application forms being either solid, e.g. powders, tablets, capsules, liquid, e.g. oral or injectable solutions; or semiliquid, e.g. salves, ointments.

For liquid preparations containing said cyclodextrin based compositions, any of the usual pharmaceutical components may be added, such as, for example, glycols, oils, alcohols and the like, however in concentrations below the level of irritation. In order to stabilize the formulations the pH may be increased or decreased or stabilized by adding appropriate acids, bases or buffer systems, e.g. citrate, phosphate buffers. Further additives may comprise substances to make the formulations isotonic, e.g. sodium chloride, mannitol, glucose and the like. It is further recommendable to add a preservative to the formulations such as, for example, a mercury salt or complex salt, e.g. phenyl mercuriacetate, nitrate, chloride or borate, phenylethyl alcohol, ethanol, propylene glycol and the like.

For solid preparations of said cyclodextrin based compositions, any of the usual solid carriers may be added to the aforementioned cyclodextrin complexes.

In the final compositions, the cyclodextrin will comprise about 2.5 to 60% by weight, in particular about 2.5 to 30%, more in particular 5 to 25%, or 5 to 20%, for example about 10%, with the remainder being water, preservative, the active ingredient and any excipients.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, injectable solutions or suspensions, and drops.

The following examples are meant to illustrate the present invention and not to limit it thereto. All percentages are by weight, unless specified otherwise.

## Examples

### Example 1

(2-Carboxymethoxy)propyl-α-cyclodextrin (I). 28.5 g (0.025 mole) of (2-hydroxy)propyl-α-cyclodextrin (MS : 2.8) were dissolved in 142 ml of water whereupon 50 g (1.25 moles) of NaOH were added. The reaction mixture was cooled to about 55-60 °C and a mixture of 18.2 g (0.193 mole) of chloroacetic acid in 13.5 ml of 40% sodium hydroxide solution was added dropwise within an hour. The reaction mixture was stirred at that temperature for 4 additional hours, then cooled to room temperature and about 100 ml of water was distilled off in vacuo. The concentrated reaction mixture was diluted with 400 ml of 96% ethanol, whereupon two layers were formed. The lower layer was washed 4 times with ethanol. The obtained oily substance was then diluted with 20 ml water and 20 ml ethanol, neutralized with 40 g of carbon dioxide. A white crystalline precipitate was formed, which was mixed in 20 ml ethanol, cooled to -30 °C and allowed to stand about 2 hours at that temperature. The precipitate was filtered off and washed twice with ethanol. Ethanol was evaporated in vacuo from the resulting yellow solution, which was acidified by adding concentrated hydrochloric acid, clarified by 2.2g of charcoal at room temperature for 30 min., filtered and washed twice with 8 ml water. The white to slightly yellow solution was then lyophylized, resulting in a very hygroscopic nearly white solid (27.9 g). This salt-containing crude product was refluxed in 82 ml of 90% ethanol for 15 min., the remaining salt was filtered off, washed twice with 47 ml hot 90% ethanol, then the ethanol was evaporated in vacuo at max. 40 °C. The solution was lyophylized, resulting in 18.6g (57.2%) of the desired compound, which contains about 1-2% of water, 2.5-3.5% of sodium chloride, acidity was about 2.8 meq./g.

### Example 2

(2-Carboxymethoxy)propyl-β-cyclodextrin (II). To a solution of 32.3 g (0.025 mole) of (2-hydroxy)-propyl-β-cyclodextrin (MS : 2.7) in 163 ml of water, there were added 50 g (1.25 moles) of sodium hydroxide. The reaction mixture was cooled to about 55-60 °C and treated with 18.2 g (0.193 mole) of chloracetic acid in 13.5 ml of 40% sodium hydroxide solution as in Example 1. The white or slightly yellow solution was subsequently lyophylized, resulting in a very hygroscopic nearly white solid (33.7 g). This salt-containing crude product was treated similarly as in Example 1, resulting in

22.3 g (61.5%) of the desired compound, which contains about 1-2% of water, 2.5-3.5% of sodium chloride, acidity was about 2.4 meq./g.

Example 3

(2-Carboxymethoxy)propyl-γ-cyclodextrin (III). 36.3 g (0.025 mole) of(2-hydroxy)propyl-γ-cyclodextrin (MS : 2.6) was dissolved in 182 ml of water and 50 g (1.25 moles) of sodium hydroxide was added. The reaction mixture was cooled to about 55-60 ° C and then there were added 18.2 g (0.193 mole) of chloracetic acid in 13.5 ml of 40% sodium hydroxide solution as in Example 1. The white to slightly yellow solution was subsequently lyophylized, resulting in a very hygroscopic nearly white solid (37.7 g). This salt-containing crude product was treated similarly as in Example 1, yielding 24.3 g (60.7%) of the desired compound, which contains about 1-2% of water, 2.5-3.5% of sodium chloride, acidity was about 2.1 meq./g.

Example 4

(2-Carboxymethoxy)propyl-(2-hydroxy)propyl-α-cyclodextrin (IV). 11.4g (0.01 mole)of (2-hydroxy)-propyl-α-cyclodextrin (DS : 2.8) was dissolved in 57 ml of water to which was added 20 g (0.5 mole) of sodium hydroxide. The reaction mixture was cooled to about 55-60 ° C and 5.2 g (0.055 mole) of chloracetic acid in 9.5 g of 40% sodium hydroxide solution were added as in Example 1. The reaction mixture was allowed to cool to room temperature and 200 ml of 96% ethanol were added. The oily phase was separated and washed twice with 10 ml of 96% of ethanol. The resulting oily material was subsequently diluted with 40 ml of water and 40 ml of ethanol, neutralized with 20 g of carbon dioxide whereupon a white crystalline precipitate was formed. The mixture was diluted with 40 ml of ethanol, cooled to -30 ° C and allowed to stand for about 2 hours at that temperature. The precipitate was filtered off and washed twice with 20 ml of ethanol. The latter was evaporated in vacuo from the resulting yellow solution, and the residue was acidified by adding concentrated hydrochloric acid, and clarified with 0.5g of charcoal at room temperature for 30 min. The mixture was filtered and washed twice with 5 ml of water. The white to slightly yellow solution was then lyophylized, resulting in a very hygroscopic nearly white solid (7.3 g). This salt-containing crude product was refluxed in 21 ml of 75% ethanol for 15 min., the remaining salt was filtered off. The latter was washed twice with 21 ml 75% ethanol, subsequently the ethanol was evaporated in vacuo at max. 40 ° C, and the solution was lyophylized, resulting in 4.9 g of the desired compound, which contains about 1-2% of water, 3.0-5.0% of sodium chloride, acidity was about 1.6 meq./g.

Example 5

Following the same procedures as in example 4 but starting from 13 g (0.01 mole) of (2-hydroxy)-propyl-β-cyclodextrin (MS : 2.7) in 65 ml of water, there was obtained 7.9 g (2-Carboxymethoxy)-propyl-(2-hydroxy)propyl-β-cyclodextrin (V) which contains about 1-2% of water, 3.0-5.0% of sodium chloride, acidity was about 1.3 meq./g.

Example 6

Following the same procedures as in example 4 but starting from 14.5 g (0.01 mole) of (2-hydroxy)-propyl-γ-cyclodextrin (MS : 2.6) in 73 ml of water, there were prepared 8.9 g of (2-Carboxymethoxy)-propyl-(2-hydroxy)propyl-γ-cyclodextrin (VI), which contains about 1-2% of water, 3.0-5.0% of sodium chloride, acidity was about 1.1 meq./g.

Example 7

(2-Carboxymethoxy)propyl-carboxymethyl-α-cyclodextrin (VII). 11.4g (0.01 mole) of (2-hydroxy)-propyl-α-cyclodextrin (MS : 2.8) was dissolved in 57 ml of water, whereto 20 g (0.5 mole) of sodium hydroxide were added. The reaction mixture was cooled to about 55-60 ° C and treated with 8.3 g (0.088 mole) of chloroacetic acid in 15.5 g of 40% sodium hydroxide solution as in Example 4. The white or slightly yellow solution was then lyophylized, yielding a very hygroscopic nearly white solid (12.3 g). This salt-containing crude product was treated similarly as in Example 4, resulting in 7.9 g of the desired compound, which contains about 1-2% of water, 0.5-1.5% of sodium chloride, acidity was about 2.0 meq./g.

Example 8

(2-Carboxymethoxy)propyl-carboxymethyl-β-cyclodextrin (VIII).
Method A: 13 g (0.01 mole) of (2-hydroxy)propyl-β-cyclodextrin (MS : 2.7) were dissolved in 65 ml of water whereto 20 g (0.5 mole) of sodium hydroxide were added. The reaction mixture was cooled to about 55-60 ° C and treated with 8.3 g (0.088 mole) of chloroacetic acid in 15.5 of 40% sodium hydroxide solution as in Example 4. The white or slightly yellow solution was then lyophylized, resulting in a very hygroscopic nearly white solid (13.2 g). This salt-containing crude product was treated similarly as in Example 4, resulting in 9.8 g of the desired compound, which contains about 1-2% of water, 0-1.5% of sodium chloride, acidity was about 1.8

meq./g. (2-Carboxymethoxy)propyl-carboxymethyl-β-cyclodextrin (IX).

Method B : 32.3 g (0.025 mole) of (2-hydroxy)-propyl-β-cyclodextrin (DS : 2.7) was dissolved in 163 ml of water, then 50 g (1.25 mole) of sodium hydroxide were added. The reaction mixture was cooled to about 55-60°C and 18.2 g (0.193 mole) of chloroacetic acid in 13.5 ml of 40% sodium hydroxide solution was added. The reaction mixture was stirred at that temperature for 4 additional hours, then cooled to room temperature and about 100 ml of water was removed by distillation in vacuo. The concentrated reaction mixture was diluted with 400 ml of 96% of ethanol. The upper layer was evaporated until its volume was reduced to about 100 ml. 100 ml of ethanol was added and about 150 g of carbon dioxide was bubbled into the solution. The formed precipitate was filtered off and washed twice with 50 ml of ethanol and subsequently 50 g of carbon dioxide was bubbled through the solution. The formed precipitate was filtered off, washed twice with 50 ml of ethanol, then another 50 g of carbon dioxide was bubbled into the solution. The formed precipitate was filtered off, washed twice with 50 ml of ethanol, then the ethanol was evaporated, and the remaining aqueous solution was acidified with concentrated hydrochloric acid. The obtained yellow solution was clarified with 5 g of charcoal at room temperature for 1 hour, filtered and washed twice with 10 ml of water, and subsequently lyophylized. The very hygroscopic material (46.7 g) was refluxed in 100 ml of 90% ethanol and the remaining insoluble material was washed three times with 30 ml of 90% ethanol; the ethanol phases were evaporated, the remaining solution was diluted with 20 ml of water and lyophylized, resulting in 11.9 g of desired material. This product contains about 4-5% of water, 2.5-4.0% of sodium chloride, acidity was about 0.7 meq./g.

Example 9

(2-Carboxymethoxy)propyl-carboxymethyl-γ-cyclodextrin (X). 14.5 g (0.01 mole) of (2-hydroxy)-propyl-γ-cyclodextrin (MS : 2.6) was dissolved in 73 ml of water, whereto 20 g (0.5 mole) of sodium hydroxide was added. The reaction mixture was cooled to about 55-60°C and treated with 8.3 g (0.088 mole) of chloroacetic acid in 15.5 g of 40% sodium hydroxide solution as in Example 4. The white or slightly yellow solution was subsequently lyophylized, resulting in a very hygroscopic nearly white solid (15.9 g). This salt-containing crude product was treated similarly as in Example 4, resulting in 11.9 g of the desired compound, which contains about 1-2% of water, 0.5-2.0% of sodium chloride, acidity was about 1.5 meq./g.

Example 10

(2-Carboxyethoxy)propyl-β-cyclodextrin (XI). 13 g (0.01 mole) of the starting material of Example 2 was dissolved in 26 ml of water, then 11.8 g (0.2 mole) of potassium hydroxide was added. The reaction mixture was cooled to room temperature and 3.9 g (0.055 mole) of acrylamide was added in one portion, stirred overnight at room temperature, then heated to 100-105°C and stirred at that temperature until the amide was fully hydrolyzed. The reaction mixture was cooled to about 0-5°C, diluted with 150 ml of 96% ethanol, allowed to separate in two layers and the oily phase was washed twice with 50 ml of water and 50 ml of ethanol, subsequently acidified with 5 N sulfuric acid, cooled to about -10°C and allowed to stand for 2 hours. The precipitate was filtered off, washed three times with 96% ethanol. Ethanol was evaporated, the aqueous solution was diluted with 45 ml of water, clarified with 1.5 g of charcoal, filtered, the charcoal was washed twice with 5 ml of water. The white or slightly yellow solution was lyophylized, resulting in 13.5 g (84%) of the desired material, which contains 1-3% of water, acidity was about 2.0 meq./g.

Example 11

(2-Carboxamidoethoxy)propyl-β-cyclodextrin (XII). 13 g (0.01 mole) of starting material of Example 2 was dissolved in 26 ml of water, whereto 11.8 g (0.2 mole) of potassium hydroxide was added. The reaction mixture was cooled to room temperature and 3.9 g (0.055 mole) of acrylamide was added in one portion, stirred for 1 hour, cooled to about 0-5°C, and neutralized with 5 N sulfuric acid. The resulting solution was lyophylized, and the residue was dissolved in a four-fold volume of dry methanol. The remaining insoluble material was filtered off and the methanol was evaporated, resulting in 12 g of crude product. This crude product was dissolved in a two-fold volume of methanol and filtered through a ten-fold of alumina, eluted with dry methanol and the fractions containing the desired compound were collected, methanol was evaporated in vacuo, subsequently lyophylized, yielding 2.1 g (14%) of a slightly hygroscopic nearly white material.

Example 12

(2-Carboxyethoxy)propyl-carboxyethoxy-β-cyclodextrin (XIII). 32.3 g (0.01 mole) of starting material of Example 2 was dissolved in 26 ml of water, whereto 11.8 g (0.2 mole) of potassium hydroxide were added. The reaction mixture was cooled to room temperature and 5.5 g (0.077 mole) of acrylamide was added in one portion, and the

mixture was stirred overnight at room temperature, then heated to 100-105 °C and stirred at that temperature until the amide was fully hydrolyzed. The reaction mixture was cooled to about 0-5 °C, diluted with 150 ml of 96% ethanol, allowed to separate and the oily phase was washed twice with 50 ml of ethanol. The obtained oil was diluted with 20 ml of water and 40 ml of ethanol and subsequently acidified with 5 N sulfuric acid, cooled to about -10 °C and allowed to stand for 2 hours. The precipitate was filtered off, washed three times with 96% ethanol. The latter was evaporated and the residue was diluted with 45 ml of water, clarified with 1.5 g of charcoal, filtered and the charcoal was washed twice with 5 ml of water. The white or slightly yellow solution was lyophylized, resulting in 13.5 g of the desired material, which contains 1-3% of water, acidity was about 3.2 meq./g.

Example 13

(3-Carboxymethoxy)propyl-β-cyclodextrin (XIV). 32.8 g (0.025 mole) of 3-hydroxy)propyl-β-cyclodextrin (DS : 3) was dissolved in 164 ml of water, whereto 8 g (0.20 mole) of sodium hydroxide were added. The reaction mixture was cooled to about 45-50 °C and treated with 14.0 g (0.15 mole) of chloroacetic acid in 10.5 ml of 40% sodium hydroxide solution as in Example 1. The white or slightly yellow solution was then lyophylized, resulting in a very hygroscopic nearly white solid (34 g). This salt-containing crude product was treated similarly as in Example 1, resulting in 20.3 g (54.6%) of the desired compound, which contains about 1-2% of water, 2.5-4.0% of sodium chloride, acidity was about 2.2 meq./g.

Example 14

(2-Carboxymethoxy)ethyl-β-cyclodextrin (XV). 31.9 g (0.025 mole) of (2-hydroxy)ethyl-β-cyclodextrin (DS : 3.1) was dissolved in 160 ml of water, whereto 8 g (0.20 mole) of sodium hydroxide were added. The reaction mixture was cooled to about 45-50 °C and treated with 14.0 g (0.15 mole) of chloroacetic acid in 10.5 ml of 40% sodium hydroxide solution as in Example 1. The white or slightly yellow solution was then lyophylized, yielding a very hygroscopic nearly white solid (37 g). This salt-containing crude product was treated similarly as in Example 1, resulting in 18.9 g (54.6%) of the desired compound, which contains about 2-3% of water, 3.5-4.5% of sodium chloride, acidity was about 2.4 meq./g.

Example 15

(2(3)-Carboxymethoxy-3(2)-hydroxy)butyl-β-cyclodextrin (XVI). 33.9 g (0.025 mole) of (2,3-dihydroxy)butyl-β-cyclodextrin was dissolved in 170 ml of water whereto 50 g (1.25 mole) of sodium hydroxide were added. The reaction mixture was cooled to about 55-60 °C and 18.2 g (0.193 mole) of chloroacetic acid in 13.5 ml of 40% sodium hydroxide solution was added dropwise during one hour. The reaction mixture was stirred at that temperature for 4 additional hours, then cooled to room temperature and about 125 ml of water was distilled off in vacuo. The concentrated reaction mixture was diluted with 400 ml of 96% ethanol, whereupon two phases arise. The lower layer was washed 4 times with ethanol. The resulted oily material was then diluted with 20 ml of water and 20 ml of ethanol, neutralized with 40 g of carbon dioxide, whereupon a white crystalline precipitate forms, diluted with 20 ml of ethanol, cooled to -30 °C and allow to stand for about 2 hours at that temperature. The precipitate was filtered off, washed twice with ethanol. Ethanol was evaporated in vacuo from the resulting yellow solution, and the residue was acidified by adding concentrated hydrochloric acid, clarified with 2.2 g of charcoal at room temperature for 30 min, filtered, washed twice with 8 ml of water. The white or slightly yellow solution was lyophylized, resulting in a very hygroscopic nearly white solid (36.8 g). This salt-containing crude product was refluxed in 110 ml of 90% ethanol for 15 min, the remaining salt was filtered off, washed twice with 55 ml of hot 90% ethanol, and the ethanol was evaporated in vacuo at max. 40 °C. The solution was lyophylized, resulting in 24.5 g (66.8%) of the desired compound, which contains about 1-2% of water, 3.0-4.0% of sodium chloride, acidity was about 2.0 meq./g.

Example 16

(2-Carboxymethoxy)propyl-β-cyclodextrin peracetate (XVII). 1.4 g (0.001 mole) of II. were dissolved in 7 ml of acetic anhydride and 7 ml of anhydrous pyridine, stirred for 24 hours at room temperature and for an additional 72 hours at 50-55 °C. After completion, the mixture was diluted with water, extracted with chloroform; after drying, the solvent was evaporated, and co-evaporated with toluene, yielding in 2.2 g (70%) of a slight yellow material.

Example 17

(2-Methoxycarbonylmethoxy)propyl-β-cyclodextrin (XVIII). 1.4 g (0.001 mole) of compound II. were dissolved in 14 ml of water and 0.24 g (0.006 mole) of sodium hydroxide, and 0.63 g (0.005 mole) of dimethyl sulfate were added. The reaction mixture

was stirred for 1 hour, neutralized, and water was evaporated by distillation at max. 40 °C in vacuo. The resulting material was refluxed in 10 ml of 96% ethanol, the solid residue was filtered off, washed twice with 4 m of ethanol and from the filtrate ethanol was evaporated. The residue was diluted with 10 ml of water and lyophylized, resulting in 1.4 g of slightly yellow material.

Example 18

(2-Methoxycarbonylmethoxy)propyl-$\beta$-cyclodextrin peracetate (XIX). 1.4 g (0.001 mole) of II. was dissolved in 14 ml of water and 0.24 g (0.006 mole) of sodium hydroxide and then 0.63 g (0.005 mole) of dimethyl sulfate was added. The reaction mixture was stirred for 1 hour, neutralized, and water was evaporated by distillation at max. 40 °C in vacuo. The resulting material was dried overnight over phosphorous pentoxide at 40 °C in vacuo and subsequently dissolved in 7 ml of acetic anhydride and 7 ml of anhydrous pyridine, stirred for 24 hours at room temperature and for an additional 72 hours at 50-55 °C. After completion, this mixture was diluted with water and extracted with chloroform. The extracts were dried, filtered and evaporated and the residue was co-evaporated with toluene, resulting in 2.1 g (70% yield) slightly yellow material.

Example 19

(2-Carboxymethoxy)propyl-$\beta$-cyclodextrin (XX). 64.6g (0.057 mole) of (2-hydroxy)propyl-$\beta$-cyclodextrin was dissolved in 326 ml of water whereupon 100 g (2.5 moles) of NaOH was added. The reaction mixture was cooled to about 50 °C and a mixture of 36.7 g (0.39 mole) of chloroacetic acid in a sodium hydroxide solution (pH = 7) was added dropwise within an hour. After the reaction mixture was stirred at that temperature for 4 hours, about 200 ml of water was distilled off in vacuo. The concentrated reaction mixture was diluted with 800 ml of 96% ethanol and cooled, whereupon two layers were formed. The lower layer was washed 3 times with ethanol at 4 °C. The obtained oily substance was then vigorously shaken with 40 ml water and 40 ml ethanol was added, whereupon a white crystalline precipitate ($NaHCO_3$) was formed. The solution was neutralized with carbon dioxide, cooled to -30 °C and allowed to stand 20 to 30 minutes at that temperature. The precipitate was filtered off and washed twice with ethanol/water (90/10). The ethanol was evaporated in vacuo at 50 °C, and the residue was diluted with water to about 200 ml. After cooling with carbon dioxide/2-propanone, the mixture was acidified by adding sulfuric acid 5 M to pH 2.5. At room temperature, the mixture was clarified by treatment with 4 g of charcoal for 30 min., filtered and washed twice with 30 ml water. The solution was then lyophylized and the resulting product was refluxed in 80 ml of 90% ethanol for 15 minutes. The remaining salt was filtered off and washed twice with 45 ml hot 90% ethanol. The ethanol was evaporated in vacuo at 40 °C and the solution was lyophylized.

Example 20

Increase of tolnaftate solubility. An excess of tolnaftate was added to aqueous solutions containing various concentrations of $\alpha$-, $\beta$- or $\gamma$-cyclodextrin derivatives according to the present invention and shaken at 25±0.5 °C. After equilibrium was attained (1-32 days), an aliquot was filtered through a membrane filter. A portion of each sample was diluted and analyzed spectrophotometrically. A substantial increase of tolnaftate solubility was observed. Similar results were obtained with dipyridamol, hydrocortisone, silver sulfadiazine, 1,3-dihydro-7-nitro-5-phenyl-2H-1,4-benzodiazepine-2-one; 1-[2-(2,4-dichlorophenyl)-2-[[(2,4-dichlorophenyl)-methyl]oxy]ethyl]imidazole; 4-[(2-thienyl)carbonyl]-$\alpha$-methylbenzeneacetic acid; cis-4-[4-[4-[4-[[2-(2,4-dichlorophenyl)-2-(1H-1,2,4-triazol-1-ylmethyl)-1,3-dioxolan-4-yl]methoxy]-phenyl]-1-piperazinyl]phenyl]-2,4-dihydro-2-(1-methylpropyl)-3H-1,2,4-triazol-3-one; and 2-[2-(4-chlorophenyl)-2-oxoethyl]-2,4-dihydro-4-[4-[4-(4-hydroxyphenyl)-1-piperazinyl]phenyl]-5-methyl-3H-1,2,4-triazol-3-one.

Example 21

Increase of stability of indomethacine in solution. A 1% solution of $\beta$-cyclodextrin derivatives according to the present invention were equilibrated with 10 mg of indomethacine for 24 hours. The resulting solutions were membrane filtered and then stored at 22 ±0.5 °C in daylight in glass vials for 9 days. The intact indomethacine content of the solutions were measured from time to time. The cyclodextrins according to the present invention significantly increase the chemical stability of the indomethacine in pH 6.6 phosphate buffer under the given conditions.

**Claims**

**1.** A cyclodextrin ether or mixed ether wherein one or several hydroxy groups of the cyclodextrin moiety are etherified with mono- or dihydroxyalkyl and in the case of mixed ethers, one or more of the remaining hydroxy groups of the cyclodextrin moiety are further

etherified with (carboxyl)alkyl or (derivatized carboxyl)alkyl, characterized in that one or more of the hydroxy groups of said mono- or dihydroxyalkyl groups are further etherified with (carboxyl)alkyl or (derivatized carboxyl) alkyl; the alkylcarbonylated derivatives thereof; the non-toxic metal and amine salt forms thereof; and the stereochemically isomeric forms thereof.

2. A cyclodextrin ether or mixed ether according to claim 1 wherein said derivatized carboxyl is an amide, a mono- or dialkyl amide, a cyclic alkyl amide or an alkyl ester.

3. A cyclodextrin ether or mixed ether according to claim 2 wherein the MS of the hydroxyalkyl or dihydroxyalkyl substituents is in the range from $\frac{1}{8}$ , $\frac{1}{7}$ or $\frac{1}{6}$ to 10.

4. A cyclodextrin ether or mixed ether according to claim 1 wherein one or several hydroxy groups of the cyclodextrin moiety are etherified with a substituent of formula

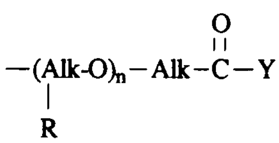 (a)

wherein R is hydrogen, hydroxy, -O-$(Alk-O)_n$-H or -O-$(Alk-O)_n$-Alk-C(=O)-Y,

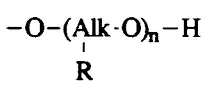

or

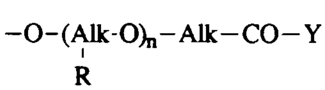 ,

and wherein R may be present on one or more of the -Alk-O- moieties;
each Alk independently is $C_{1-4}$alkanediyl; and where Alk is substituted with R, Alk is $C_{2-4}$alkanetriyl;
each n independently is the integer 1 to 10; Y is $OR^1$ or $NR^2_2$ , wherein $R^1$ and $R^2$ each independently are hydrogen or $C_{1-4}$alkyl, or both $R^2$ combined with the nitrogen bearing these substituents may form a morpholinyl, piperidinyl, pyrrolidinyl or piperazinyl ring;
and one or more of the remaining hydroxy groups of the cyclodextrin moiety may be further etherified with a radical -$(Alk-O)_n$-H or -Alk-CO-Y; the alkylcarbonylated derivatives, salts and stereoisomers thereof.

5. A cyclodextrin ether or mixed ether according to claim 4 wherein the radical of formula (a) is:

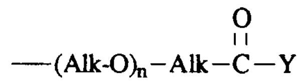 ,

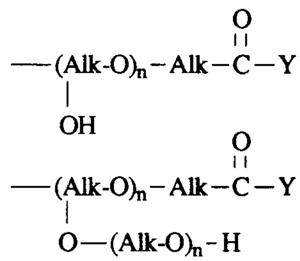 , or

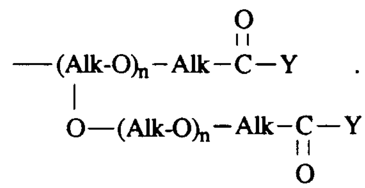 .

6. A process for preparing a cyclodextrin ether or mixed ether as claimed in any of claims 1 to 5, characterized by reacting a cyclodextrin ether or mixed ether wherein one or several hydroxy groups of the cyclodextrin moiety are etherified with mono- or dihydroxyalkyl and in the case of mixed ethers, one or more of the remaining hydroxy groups of the cyclodextrin moiety are further etherified with (carboxyl)-alkyl or (derivatized carboxyl)alkyl, with a (leaving group substituted) $C_{1-4}$alkyl carboxylic acid or acid derivative or with a $\alpha$, $\beta$-$C_{2-4}$alkenyl carboxylic acid or acid derivative in the presence of a base, in an aqueous solution or in an organic solvent suspension.

7. A cyclodextrin derivative as claimed in any one of claims 1 to 5 obtainable by the process according to claim 6.

8. A complex comprising a chemical substance and a cyclodextrin derivative as claimed in any one of claims 1 to 5 or 7.

9. A complex according to claim 8 wherein said chemical substance is a drug.

10. A pharmaceutical composition comprising a complex according to claim 9 and a carrier.

European Patent Office

# EUROPEAN SEARCH REPORT

Application Number

EP 92 20 0341

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | CHEMICAL ABSTRACTS, vol. 92, no. 3, 21 January 1980, Columbus, Ohio, US; abstract no. 22773F, FOLDI ZOLTAN ET AL.: 'CARBOXYALKYL-CYCLODEXTRIN SALTS' page 711 ; * abstract * | 1-10 | C08B37/16<br>A61K47/48 |
| Y | CHEMICAL PATENTS INDEX, BASIC ABSTRACTS JOURNAL Section Ch, Week 8642, 10 December 1986 Derwent Publications Ltd., London, GB; Class A, AN 275581/42 & JP-A-61 200 965 (YAMANOUCHI PHARM KK) 5 September 1986 * abstract * | 1-10 | |
| A | ANALYTICAL CHEMISTRY. vol. 62, no. 9, 1 May 1990, COLUMBUS US pages 914 - 923; DANIEL W. ARMSTRONG ET AL.: 'POLAR-LIQUID,DERIVATIZED CYCLODEXTRIN STATIONARY PHASES FOR THE CAPILLARY GAS CHROMATOGRAPHY SEPARATION OF ENANTIOMERS' | | TECHNICAL FIELDS SEARCHED (Int. Cl.5 ) |
| A | EP-A-0 300 526 (JANSSEN PHARMACEUTICA) | | C08B<br>A61K |
| D,A | EP-A-0 146 841 (HOECHST) | | |
| A | PATENT ABSTRACTS OF JAPAN vol. 13, no. 11 (C-558)11 January 1989 & JP-A-63 218 663 ( BAYER ) 12 September 1988 * abstract * | | |

The present search report has been drawn up for all claims

| Place of search | Date of completion of the search | Examiner |
|---|---|---|
| THE HAGUE | 25 MAY 1992 | LENSEN H.W.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)